# EUROPÄISCHE PATENTANMELDUNG

(11) **EP 2 181 680 A1**
(43) Veröffentlichungstag der Anmeldung: **05.05.2010**
(21) Anmeldenummer: 09173994.6
(22) Anmeldetag: 24.10.2009
(51) Int. Cl.: A61F 11/00, A61F 11/08, A61M 31/00

(54) **Mittel zur Applikation von Wirkstoffen im Gehörgang eines Patienten**

(30) Priorität: 03.11.2008 CH 17172008
(71) Anmelder: Illi, Oskar E., 3123 Belp (CH); Feldmann, Clarence P., 8152 Opfikon (CH)
(72) Erfinder: Illi, Oskar E., 3123 Belp (CH); Feldmann, Clarence P., 8152 Opfikon (CH)
(74) Vertreter: Feldmann, Clarence Paul

(57) **Zusammenfassung**

Um Medikamente in der Form von in flüssiger oder pastöser Matrix gebundenen Wirkstoffen im Gehörgang zu applizieren wird ein neuartiges Mittel (1) vorgeschlagen, das ein Medikament aufzunehmen und lokal abzugeben vermag. Dieses besteht aus einem sackförmigen Aufnahmeteil (2), in welchen ein vorzugsweise textiler Tampon, der das Medikament aufzunehmen vermag, applizierbar ist, und einer gummielastischen Haltescheibe (3), die zur Fixierung des Aufnahmeteils (2) im äusseren Ohr dient. Das Aufnahmeteil (2) ist über die Haltescheibe (3) fest mit einem Zugorgan (4), z.B. einer Schnur verbunden. Die Haltescheibe (3) dient der Fixierung des Aufnahmeteils (2) im äusseren Gehörgang und stützt sich dabei einerseits hinter der Anthelix (106) und andererseits hinter dem Tragus (108) ab. In einer bevorzugten Ausführungsform wird die Haltescheibe (3) aus einem flachen aus Moosgummi gestanzten Material gefertigt. Der Applikator kann medikamentös beladen in den Handel gebracht werden.

## Beschreibung

Die vorliegende Erfindung betrifft ein Mittel zur Applikation von in flüssiger Matrix gebundenen Wirkstoffen im Gehörgang eines Patienten gemäss Oberbegriff des Patentanspruches 1.

Bei vielen Erkrankungen im Bereich des äusseren Gehörganges, des Trommelfelles oder des Mittelohrs (bei Perforationen des Trommelfells) werden Medikamente mit Wirkstoffen, die in flüssiger oder pastöser Matrix gebunden sind, im äusseren Gehörgang appliziert. Während Medikamente auf flüssiger Basis meist als sogenannte Ohrentropfen direkt in den äusseren Gehörgang eingeträufelt werden, werden die Medikamente auf pastöser Basis heute üblicherweise in Gestalt eines Tampons im äusseren Gehörgang appliziert. Diese Applikation erfolgt nicht durch den Patienten selber, sondern wird von einem behandelnden Arzt durchgeführt. Hierbei präpariert er üblicherweise einen Gazestreifen, den er mit dem gewünschten Medikament tränkt, danach zu einem Tampon wickelt und diesen im äusseren Gehörgang appliziert. Normalerweise braucht der Patient mehrere Tampons bis zur vollständigen Ausheilung. Dies bedingt automatisch mehrere Arztbesuche.

Die Applikation von flüssigen Medikamenten in den Gehörgang erfolgt heute meist durch Selbstverabreichung. Die Selbstverabreichung ist jedoch keineswegs problemlos. Hierzu sind beispielsweise Träufelfläschchen auf dem Markt erhältlich, die mit einem entsprechenden Ausguss versehen sind, dank dem eine tropfenweise Abgabe möglich ist. Für eine Selbstverabreichung des Medikamentes sind Träufelfläschchen höchst ungeeignet. Der Patient muss dazu das Fläschchen in der richtigen Lage distanziert über die Öffnung des Gehörganges halten und dabei nach Gefühl die korrekte Anzahl Tropfen verabreichen. Dies muss berührungsfrei erfolgen, da er ansonsten ein unkontrolliertes Ausfliessen bewirkt. Hält er jedoch das Träufelfläschchen distanziert, so ist nicht gewährleistet, dass die Tropfen am richtigen Ort appliziert werden. Ferner ist eine solche Verabreichung praktisch nur im Liegen möglich.

Des weiteren sind Medikamentenfläschchen auf dem Markt erhältlich, deren Verschluss mit einer Pipette versehen ist. Die Pipette enthält im gefüllten Zustand üblicherweise eine zu grosse Menge des flüssigen Medikamentes. Entleert der Patient somit die Pipette vollständig, so hat er eine Überdosis. Mit Fremdhilfe lässt sich mittels einer Pipette problemlos das Medikament tropfenweise abgeben, doch ist dies bei einer Selbstverabreichung kaum möglich. Berührt man mit der Pipette das Ohr, so läuft das Medikament praktisch augenblicklich vollständig aus. Will man jedoch das Medikament ohne Berührung der Pipette mit dem Ohr tropfenweise entleeren, hat man das Problem, dass man praktisch kaum richtig zielen kann. Die Tropfen gelangen somit irgendwo in die Ohrmuschel und die Wahrscheinlichkeit, dass ein Teil der applizierten Menge des Medikamentes gar nie in den Gehörgang hinein läuft, ist entsprechend gross. Die Folge ist eine Unterdosierung des Medikamentes. Ferner sind auf dem Markt Fläschchen vorhanden mit einer Gummitülle, wobei durch Quetschen der Tülle das Medikament tropfenweise abgebbar ist. Auch bei dieser Lösung bleibt die Problematik dieselbe.

Bleibt der Patient nach der Verabreichung von Ohrentropfen nicht auf derselben Seite liegen, so fliessen die verabreichten Ohrentropfen wieder aus. Dies geschieht auch wenn er sich von der horizontalen in die vertikale Lage begibt. Dies ist einerseits für den Patienten unangenehm und zum andern entsteht eine medikamentöse Unterversorgung.

Es sind aus dem Stand der Technik, jedoch nicht auf dem Markt, Mittel zur Applikation von in flüssiger und pastöser Matrix gebundenen Wirkstoffen im Gehörgang eines Patienten bekannt. So beschreibt die DE-U-200 13 494 einen Medikamententräger aus saugfähigem Material, welcher geeignet sein soll zur Einführung in einen Gehörgang.

Solche tamponähnlichen Medikamententräger aus saugfähigem Material zeigen auch die US 4 249 531, die DE-B 1 096 545, die WO 2007/03009 und die US 2 587 035, wobei das letztgenannte Dokument lediglich einen Gehörschutz beschreibt, der aber ansonsten die Merkmale des Oberbegriffes des Patentanspruches 1 aufweist.

Letztlich zeigt das deutsche Gebrauchsmuster DE-U 90 13 058 einen Applikator aus Kunststoff, der einen hohlen, geschlossenen Zapfen offenbart, der mit membranförmigen Vertiefungen versehen ist und mit einem Balg kommuniziert mittels den pastösen Medikamenten, die in den Vertiefungen haften in einen Gehörgang einführbar sind, worauf durch Betätigung des Belages die Vertiefungen nach aussen wölbt kann und das Medikament im Gehörgang abstreift.

Versuche haben gezeigt, dass saugfähige Mittel zur Applikation in flüssiger Matrix gebundenen Wirkstoffe nicht universell verwendbar sind. Die Matrix ist nämlich sehr unterschiedlich in der Viskosität und kann auf wässriger oder öliger Basis aufgebaut sein. Je nach Materialwahl des saugfähigen Mittels findet diese Saugwirkung eben nur bei einer wässrigen oder nur bei einer öligen Matrix statt.

Auch die Befüllung eines sackartigen Aufnahmeteiles ist nicht unproblematisch. In einfachster Form lässt sich dies durch einen Zapfen realisieren mit dem das Aufnahmeteil zu öffnen und zu schliessen ist. Bedenkt man jedoch dass der Innendruchmesser des Zapfens nur etwa 2 - 3 mm gross ist, so ist das Betätigen eines solchen Miniaturzapfens eine Forderung der viele ältere Patienten kaum gewachsen sind und insbesondere die Gefahr diese losen Zapfen zu verlieren ist sehr gross. Hinzu kommt, dass bei Medikamenten auf öliger Basis die wie ein Gleitmittel wirken auch jüngere Patienten Probleme habe einen solchen Zapfen einzusetzen. Hinzu kommt, dass auch der Zapfen kaum noch hält und die Gefahr, dass dieser herausrutscht und sogar in den Gehörgang gerät ist leider vorhanden.

Es ist daher die Aufgabe der vorliegenden Erfindung ein verbessertes Mittel zu schaffen, welches zur Applikation von in flüssiger Matrix gebundenen Wirkstoffen im Gehörgang eines Patienten geeignet ist, unter Meidung der vorgenannten Probleme.

Diese Aufgabe löst ein Mittel mit den Merkmalen des Patentanspruches 1. Weitere vorteilhafte Ausgestaltungsformen gehen aus den abhängigen Ansprüchen hervor und deren Bedeutung und Wirkungsweise ist in der nachfolgenden Beschreibung unter Bezug auf die beiliegenden Zeichnungen erläutert. In der Zeichnung ist eine bevorzugte Ausführungsform dargestellt und nachfolgend beschrieben. Es zeigt:
- Figur 1: zeigt einen achsialen Längsschnitt durch ein erfindungsgemässes Mittel und
- Figur 2: dasselbe Mittel um 90° um die Längsachse gedreht, jedoch mit einer modifizierten Füllöffnung.
- Figur 3: zeigt in vereinfachter Darstellung das erfindungsgemässe Mittel appliziert im äusseren Gehörgang in einem anatomischen Schnitt und
- Figur 4: die Verwendung desselben Mittels in einer Sicht auf das Ohr eines Anwenders.

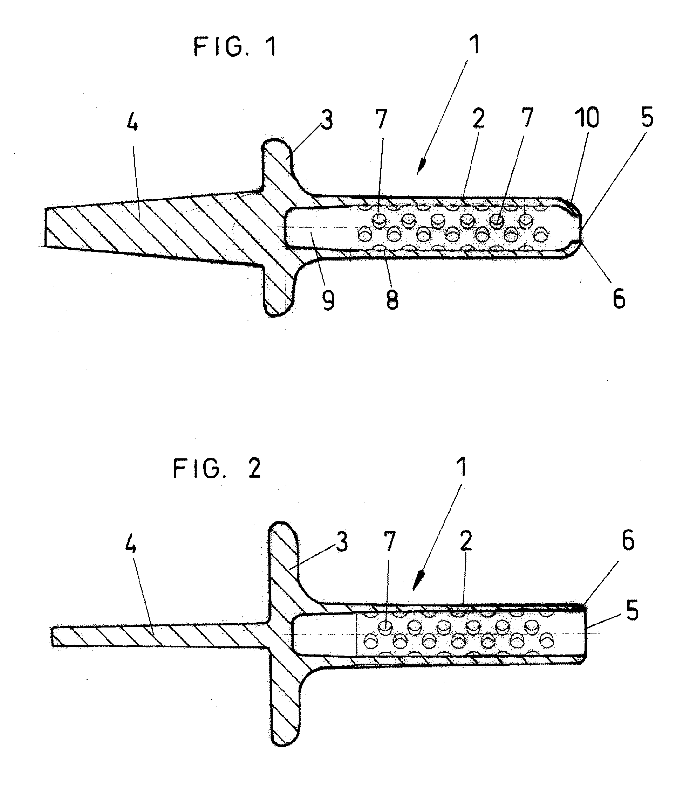

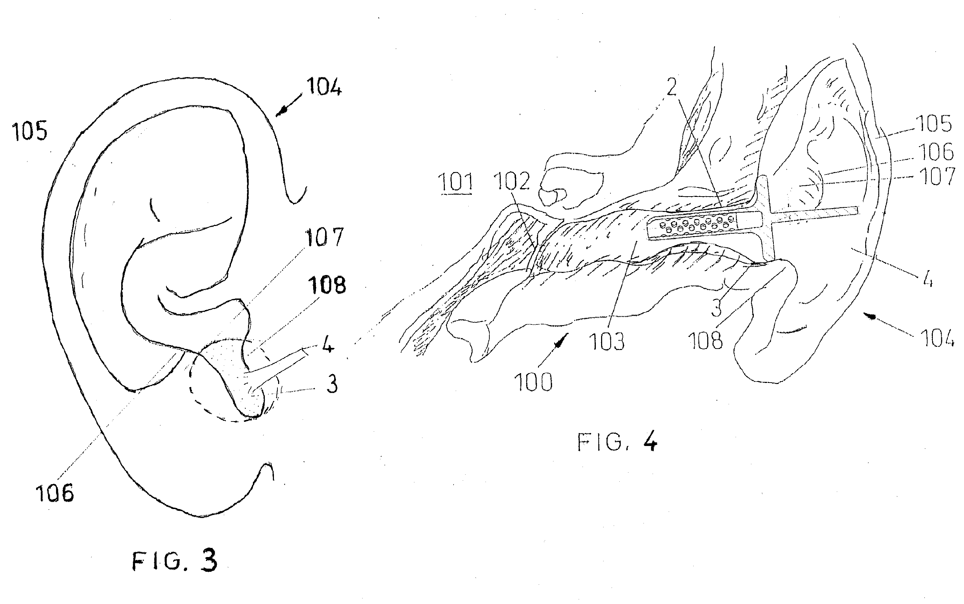

Vorerst wird mit Bezug auf die Figuren 1 und 2 auf zwei verschiedene bevorzugte Ausführungsformen des Erfindungsgegenstandes eingegangen und nachher deren Benutzung an Hand der Figuren 3 und 4 erläutert.

Das erfindungsgemässe Mittel zur Applikation von in flüssiger Matrix gebundenen Wirkstoffen im Gehörgang eines Patienten ist in den nachfolgenden beiden Figuren 1 und 2 im Detail dargstellt. Das Mittel selbst ist insgesamt mit 1 bezeichnet. Dieses Mittel 1 besteht aus einem sackartigen Aufnahmeteil 2, eine diesen an einem Ende verschliessende Haltescheibe 3 und einem an die Haltescheibe 3 angeformten Lappen als Zugorgan 4. Diese drei Hauptelemente des Mittels 1 sind allesamt einstückig spritzgusstechnisch aus Kunststoff hergestellt.

Das sackartige Aufnahmeteil 2 weist über seine zylindrische Mantelwand 8 gleichmässig verteilt eine Vielzahl von Durchbrüchen 7 auf. Diese Durchbrüche 7 sind bevorzugterweise kreisrunde Öffnungen welche die zylindrische Mantelwand 8 durchsetzen. Die Durchbrüche 7 sind in Reihen angeordnet und jeweils in zwei benachbarten Reihen um eine halbe Distanz versetzt angeordnet.

Der Innenraum 9 des sackartigen Aufnahmeteiles 2 wird an einem Ende von einer Haltescheibe 3 verschlossen. Die Haltescheibe 3 besitzt einen Durchmesser, der wesentlich grösser ist als der Durchmesser des zylindrischen, sackartigen Aufnahmeteiles 2. Diese Haltescheibe 3 dient dazu hinter dem Tragus 108 und der Concha 107 das Mittel 1 im äusseren Gehörgang 103 zu halten. Diesbezüglich wird auf die späteren Ausführungen im Zusammenhang mit der Beschreibung der Figuren 3 und 4 verwiesen. Im einfachsten Fall kann diese Haltescheibe 3 kreisrund gestaltet sein. Da das äussere Ohr 100 bei den verschiedenen Patienten in der Formgebung und Grösse variiert, ist es jedoch von Vorteil die Haltescheibe 3 oval zu gestalten, so das durch Drehen des Mittels 1 um dessen eigene Längsachse es immer möglich sein wird die Halteplatte 3 im äusseren Ohr 100 entsprechend zu fixieren. Da die Halteplatte 3 direkt am äusseren Ohr anliegt, ist es sinnvoll hier entsprechende scharfe Kanten zu vermeiden. Entsprechend wird man die Anspritzstelle des Mittels 1 nicht in den Bereich des sackartigen Aufnahmeteiles 2 oder der Haltescheibe 3 legen, sondern möglichst am freien Ende des Zugorganes 4. Dieser Teil kommt mit Sicherheit nicht in Berührung mit dem äusseren Ohr.

Das bereits erwähnte Zugorgan 4 ist hier als Lappen gestaltet mit einer insgesamt trapezförmigen Gestalt. Der Lappen 4 kann relativ dünnwandig gestaltet werden, da die erforderlichen Kräfte äusserst gering sind um das Mittel 1 in den äusseren Gehörgang 103 einzuschieben beziehungsweise herauszuziehen. Lediglich zum Verdrehen des Mittels 1 ist eine gewisse Steifigkeit des Zugorganes 4 von Vorteil.

In der Figur 1 erkennt man die Einfüllöffnung 5 deren Durchmesser kleiner ist als der Innendurchmessers des Innenraumes 9 des sackartigen Aufnahmeteiles 2. Entsprechend ist eine Einschnürung 10 der zylindrischen Mantelwand 8 im Bereich der Einfüllöffnung 5 erkennbar. Die Einfüllöffnung 5 ist mit einer scharfen Kante 6 versehen. Diese scharfe Kante 6 dient dazu die Oberflächenspannung eines Tropfens des einzufüllenden Medikamentes zu zerstören, so dass der Tropfen leichter in den Innenraum 9 gelangt. Es hat sich erwiesen, dass auf diese Weise praktisch alle otologischen Medikamente in flüssiger Form relativ einfach in den Aufnahmeteil 2 sich einbringen lassen. Versuche haben auch gezeigt, dass durch die Durchbrüche 7 ein flüssiges Medikament eindringt wenn man mit einer Pipette über die zylindrische Mantelwand 8 des Aufnahmeteiles 2 streicht. Vermutlich durch einen kapillaren Effekt der Durchbrüche 7 strömt das flüssige Medikament in den Innenraum 9. Daher kann auch die Einfüllöffnung 5 entsprechend gegenüber dem Durchmesser des Innenraumes 9 verkleinert sein. Da aber diese Einfüllmethode ungewöhnlich ist und für verschiedene Patienten nicht direkt erkennbar, ist es sinnvoll und vorteilhaft die Einfüllöffnung 5 relativ gross zu gestalten. Würde man diese Öffnung völlig schliessen, so findet aber auch dieser kapillarähnliche Effekt nicht statt, so dass die Einfüllöffnung 5 auf jeden Fall erforderlich scheint.

Wie bereits erwähnt werden von der Logik her praktisch die Mehrzahl der Patienten ohne auf einen Bedienungshinweis zu achten ein Medikament mittels einer Pipette bevorzugterweise über die Einfüllöffnung 5 einbringen. Um dies zu erleichtern besteht eine bevorzugte Ausführungsform gemäss der Figur 2 darin, dass die Einfüllöffnung 5 im Durchmesser gleich dem Innendruchmesser des Innenraumes 9 im Bereich dieser Einfüllöffnung 5 entspricht. Auch hier ist wiederum die Einfüllöffnung 5 mit einer scharfen Kante 6 versehen um die Oberflächenspannung entsprechender Tropfen zu zerstören.

In den Figuren 3 und 4 ist die Verwendung des erfindungsgemässen Mittels dargestellt. Das gesamte äussere Ohr ist mit 100 bezeichnet. Das im Detail nicht weiter dargestellte innere Ohr ist mit 101 gekennzeichnet. Die Trennung zwischen innerem Ohr 101 und äusserem Ohr 100 erfolgt durch das Trommelfell 102. Das Trommelfell begrenzt den äusseren Gehörgang 103. Die Ohrmuschel 104 wird begrenzt durch die Helix 105, den sogenannten Ohrmuschelrand. Etwa parallel zur Helix verläuft eine Bogenwulst, die Anthelix 106. Der Anthelix 106 gegenüber ist ein Knorpelvorsprung vor der äusseren Gehörgangsöffnung vorhanden. Dieser Knorpelvorsprung wird als Tragus 108 bezeichnet. Anthelix 106 und Tragus 108 liegen am Rand der Concha 107, die eine in den äusseren Gehörgang überleitende Vertiefung darstellt. Obwohl die Form und der Querschnitt des äusseren Gehörganges 103 relativ unterschiedlich sind, wird man mit nur einer Länge des Aufnahmeteils auskommen. Die weitere Anpassung am Ohr erfolgt durch die Flexibilität der Haltescheibe 3 einerseits und durch die ovale Gestalt der Haltescheibe 3 andererseits, die eine relativ grosse Anpassung erlaubt und durch Verdrehen des Mittels 1 die Halescheibe 3 sich hinter der Concha 107 und dem Tragus 108 und der Anthelix 106 zu halten vermag. In der Mehrzahl aller Fälle wird man ein Mittel gemäss der Erfindung verwenden können. Lediglich bei weniger als 1% aller Patienten ist im Prinzip eine vergrösserte Concha vorhanden, während gleichzeitig der Tragus 108 nicht oder nur sehr rudimentär vorhanden ist. Da zudem bei allen Unterkieferbewegungen der Querschnitt des äusseren Gehörganges verändert wird, wird somit die Medikamentenabgabe begünstigt.

Die erfindungsgemässen Applikatoren lassen sich ohne Medikamente direkt an Otologen und Patienten abgeben, die jeweils den Aufnahmeraum mit dem Medikament versehen.

### Liste der Bezugszahlen

- 1: Mittel zur Applikation
- 2: sackartiges Aufnahmeteil
- 3: Haltescheibe
- 4: Zugorgan
- 5: Einfüllöffnung
- 6: scharfe Kante
- 7: Durchbrüche
- 8: Mantelwand
- 9: Innenraum
- 10: Einschnürung

- 100: äusseres Ohr
- 101: inneres Ohr
- 102: Trommelfell
- 103: äusserer Gehörgang
- 104: Ohrmuschel
- 105: Helix
- 106: Anthelix
- 107: Concha
- 108: Tragus

## Patentansprüche

1. Mittel zur Applikation von in flüssiger Matrix gebundenen Wirkstoffen im Gehörgang eines Patienten, wobei das Mittel (1) aus Kunststoff spritzgusstechnisch gefertigt ist ein sackartiges Aufnahmeteil (2) mit durchbrochener Struktur zur Aufnahme der gebundenen Wirkstoffe umfasst und mit einer Haltescheibe (3) versehen ist, die zur Halterung des Mittels (1) im Gehörgang die Concha (107) und/oder Tragus (108) zu hintergreifen vermag, und einem Zugorgan (4), welches mit dem Aufnahmeteil (2) verbunden ist, **dadurch gekennzeichnet, dass** das Mittel aus Kunststoff spritzgusstechnisch gefertigt ist und das Aufnahmeteil (2) von der Haltescheibe (3) geschlossen ist und an der, der Haltescheibe (3) gegenüberliegenden Seite durch eine erweiterte Füllöffnung füllbar ist.

2. Mittel nach Anspruch 1, **dadurch gekennzeichnet, dass** das Zugorgan (4) einstückig zentrisch an der Haltescheibe (3) angeformt ist und die Form eines trapezförmigen flexiblen Plättchens besitzt.

3. Mittel nach Anspruch 1, **dadurch gekennzeichnet, dass** die Füllöffnung mindestens eine Weite besitzt, die dem halben Innendurchmesser des sackartigen Aufnahmeteiles (2) entspricht.

4. Mittel nach Anspruch 1, **dadurch gekennzeichnet, dass** die Weite der Füllöffnung genau dem Innendurchmesser des sackartigen Aufnahmeteiles (2) entspricht.

5. Mittel nach Anspruch 1, **dadurch gekennzeichnet, dass** die Füllöffnung scharfkantig gestaltet ist.

6. Mittel nach Anspruch 1, **dadurch gekennzeichnet, dass** die Haltescheibe (3) oval gestaltet ist.
